# EUROPEAN PATENT SPECIFICATION

(11) **EP 3 257 464 B1**
(45) Date of publication and mention of the grant of the patent: **30.09.2020**
(21) Application number: 16174336.4
(22) Date of filing: 14.06.2016
(51) Int. Cl.: A61B 46/10, A61B 34/30, A61B 10/00

(54) **DISPOSABLE APPARATUS AND DEVICE WITH UNSTERILE REUSABLE APPARATUS FOR STERILE APPLICATION OF A LIQUID**
EINWEGVORRICHTUNG UND VORRICHTUNG MIT NICHTSTERILER WIEDERVERWENDBARER VORRICHTUNG FÜR STERILES AUFTRAGEN EINER FLÜSSIGKEIT
APPAREIL JETABLE ET DISPOSITIF AVEC APPAREIL RÉUTILISABLE NON STÉRILE POUR APPLICATION STÉRILE D'UN LIQUIDE

(43) Date of publication of application: 20.12.2017
(60) Divisional application: 20185406.4
(73) Proprietor: RenovaCare Sciences Corp., New York, NY 10022 (US)
(72) Inventor: SCHUBERT, Frank, 10242 Berlin (DE); BHOGAL, Jatinder S., Vancouver, BC V6B 1P1 (CA); BOLD, Thomas, 12049 Berlin (DE)
(74) Representative: Pfenning, Meinig & Partner mbB

(56) References cited:
- WO-A1-2013/075204
- WO-A1-2015/142814
- WO-A2-2009/061915
- DE-A1-102014 117 407
- US-A1- 2009 248 039
- US-A1- 2013 274 609
- US-A1- 2014 343 454

## Description

The invention relates to a disposable apparatus for sterile handling of an unsterile reusable apparatus.

The disposable apparatus comprises a film with a sterile upper side and an underside and at least one adaptor with a sterile upper side and an underside, wherein the sterile upper side of the adaptor is suitable for receiving a sterile application device. The apparatus is characterised in that the underside of the adaptor forms an irreversible and air-tight connection with the upper side of the film which is suitable for protection from contamination of the upper side of the film by contaminants originating from the underside of the film. Importantly, the underside of the adaptor has at least one anchoring point for establishing a reversible mechanical connection to an unsterile reusable apparatus. When connected to the unsterile reusable apparatus, the apparatus prevents that contaminants can reach the sterile upper side of the adaptor and upper side of the film. Therefore, contamination of a sterile application device connected to the inventive apparatus via the adaptor is prevented. In addition, a device for sterile application and a method for sterile covering of unsterile reusable apparatus are presented.

Disposable sterile covers are of interest for many innovative medical pieces of equipment which have electrical, electromechanical and/or electronic components. Sterilisation of biomedical equipment can be a challenge if the latter comprises electrical or electronic components because these pieces of equipment must not be exposed to liquids used for cleaning or sterilisation, steam and/or other methods (heat effect) or substances used for cleaning or sterilisation. For example it concerns the following pieces of medical equipment:
a) equipment which is intended to distribute an irrigation solution for wound cleaning by means of a spray head in a tissue-conserving manner (e.g. unsterile medical spray equipment with a sterile disposable spray head which keeps a wound moist or can clean it);
b) equipment which is intended to apply a debridement solution for wound debridement abrasively, by means of a pump at high pressure, on a wound (e.g. unsterile pump apparatus with a sterile disposable spray head for surgical spray debridement or cutting equipment for medical split skin production with an unsterile drive part and a sterile disposable cutting component); and/or
c) spray equipment which is intended to distribute a solution from a medical Luer lock syringe, by means of a motor-driven piston pump, uniformly over a wound.

The equipment concerned comprises typically disposable components which must be held and operated by the user with sterile gloves and components for reuse (reusable components, such as e.g. equipment main elements). The reusable components comprise electrical, electronic or other sensitive components in their interior. Sterilisation thereof is consequently associated with the risk of irreversibly destroying these sensitive components.

Spraying of cells is of interest for distribution of cell suspensions on a specific surface (e.g. on a tissue wound). The spraying of cells is used for example in surgery in order to regenerate traumatised tissue. Skin flap grafting belongs in this field for the purpose of treating skin defects. In general, this type of graft is characterised according to the recipient-donor relationship thereof. The grafting used most frequently clinically is "autologous" grafting, skin being removed from one region of the body and transferred to another region of the body of the same patient. Grafted tissue which is intrinsic to the body then develops its own blood supply at the target location and is connected finally to the tissue at the graft site. At present, several variants of autologous skin grafting are used, amongst those full skin- and split- and mesh skin grafting. Each of these grafting methods must be prepared with a specific technique and has its implicit advantages and disadvantages.

One method for obtaining tissue is *in vitro* culture of epidermal cells for cell-based wound covering ("cell sheet"). Firstly, suitable skin components, such as for instance epithelial cells, determined autologously, are obtained from the patient, which cells are subsequently increased (reproduced) and prepared *in vitro.* An autologous epithelial cell graft cultivated in this way is provided in coherently grown cell complexes (sheet). This development represents an important addition in the covering of fairly large-area skin wounds, e.g. burns. The advantage of this methodology resides in the capacity of cells to achieve coherent cell complexes which are suitable as so-called cell sheet for grafting. Hence, the methodology circumvents many disadvantages of the previously described "extensive" skin grafting methods, for instance by reducing the requirement for donor skin. However, these cell complexes cultivated *in vitro* grow relatively slowly, which is contrary to the desire for wound covering as rapidly as possible of the wound area of the recipient to be treated.

As an alternative, the use of individual cells, i.e. cells outside cell complexes, is described. These cells are present suspended in a solution and are applied in this suspension on the skin wound (see Navarra et al. (2000) J. Burn Care & Rehabilitation, 21:513-518 and Wood et al. (2003) J. Wounds, 15:16-22). The cell-suspension can be distributed in small quantities over the grafting area via pipetting or dropping, injection syringe or similar instruments. Electrically operated and electronically controlled spray devices, in contrast, can allow for controlled application of such cells on wound surfaces. A manual cell sprayer can be sterilised without difficulty since it comprises no electrical or electronic components. In contrast, automatic cell sprayers have a motor drive, a battery and similar sensitive components, such as e.g. electrical wires, which can be damaged irreversibly during the sterilisation process by the effect of water, disinfectants and/or heat.

In "operation sterile technique", steam-sterilised surgical sheets are wrapped around a non-sterilisable piece of equipment such that the user of the equipment (e.g. surgeon) can operate the unsterile equipment through the sheet in a sterile manner. Typically, merely a plastic material tube is hereby pushed over a portion of the equipment, whilst the electronic portion of the equipment is handled by the surgeon in an unsterile manner and his operating gloves thereby remain unsterile. If the operating gloves of the surgeon are intended to remain sterile, the entire electronic equipment must be sterilised before the operation although the above-mentioned disadvantages are associated therewith.

US 2014/0343454 A1 discloses an apparatus and a method for removing biopsy specimen from a bone and/or associated bone marrow using a powered drive and an intraosseous needle set. WO 2015/142814 A1 discloses a cannula mount for a surgical system which may include a body having an aperture for receiving a portion of the cannula and a block having a cam surface for engaging a cam follower surface of a clamping arm. US 2009/0248039 A1 discloses a robotic surgical system including a sterile surgical instrument, a robotic surgical manipulator, and a sterile drape covering at least a surface of the robotic surgical manipulator. WO 2009/061915 A2 discloses a sterile adaptor, a sterile drape with the integrated sterile adaptor and a telerobotic surgical system including the sterile drape. US 2013/0274609 A1 discloses pleated bags used with interventional pullback systems like e.g. imaging catheters.

It was the object of the present invention to provide a disposable apparatus which make it possible to handle an unsterile reusable apparatus in a sterile manner without the reusable apparatus requiring to be sterilised in advance.

The object is achieved by the disposable apparatus according to claim 1 and the device for sterile application according to claim 11. The dependent claims reveal advantageous developments.

According to the invention, a disposable apparatus for sterile handling of an unsterile reusable apparatus is provided, comprising
a) a film with a sterile upper side and an underside;
b) at least one adaptor with a sterile upper side and an underside, wherein the sterile upper side of the adaptor is suitable for receiving a sterile application device and wherein the underside of the adaptor has at least one anchoring point for establishing a reversible mechanical connection to an unsterile reusable apparatus;
wherein the underside of the adaptor forms an irreversible and air-tight connection with the upper side of the film,
characterized in that the adaptor comprises a mounting for a sterile application device or consists thereof, wherein the mounting is a syringe mounting and/or spray solution container mounting.

The disposable apparatus can be connected reversibly to an unsterile reusable apparatus by means of the adaptor. When combining the two pieces of apparatus, the unsterile reusable apparatus is protected by the disposable apparatus. It is aspired that no contaminants (microorganisms and viruses) can reach the sterile upper side of the film and the sterile upper side of the adaptor. Possible contamination of the sterile upper side of the adaptor and of the sterile application devices connected there is therefore prevented. The reusable apparatus can remain unsterile as "main equipment" and is usable in a sterile manner in a surgical operating theatre by the use of the disposable apparatus according to the invention, even without preceding sterilisation. Sterilisation of the "main equipment" is therefore no longer necessary.

The use of the disposable apparatus as a cover for the "main equipment" instead of sterilisation of the "main equipment" is more time-efficient, does not consume any energy or chemicals for the sterilisation and avoids damage to the often expensive, reusable equipment. As a result, the inventive disposable apparatus is more economic and ecological than sterilisation. It is preferred that the sterile film of the inventive disposable apparatus allows the operator to grasp and/or manipulate an unsterile item located on the underside of the film. In this regard, it is beneficial if the film is a flexible film. It is also beneficial if the film is at least partially transparent for visible light.

The disposable apparatus according to the invention is characterised in that the adaptor comprises a mounting for a sterile application device or consists thereof, wherein the mounting is a syringe mounting and/or spray solution container mounting, preferably a syringe mounting for disposable syringes and/or a spray solution container mounting for surgical cell spraying equipment.

The film of the disposable apparatus can have a flat or tubular configuration. Optionally, it has a tubular configuration and is closed on one side of the tube, preferably on the front-side of the tube. In the flat configuration, it is preferred if the film has a length and breadth which corresponds to at least twice the height of the unsterile reusable apparatus to be connected (and to be covered). In the tubular configuration, the film has a length and breadth which is suitable to fully enclose the unsterile reusable apparatus to be connected (and to be covered).

In a preferred embodiment, the film has, at least in regions, on the upper side and/or the underside, a surface which
a) is self-adhesive (i.e. has a sticky surface); and/or
b) is provided with adhesive; and/or
c) is folded, preferably is folded like a fan.

Particularly preferably, the mentioned self-adhesive surface and/or the surface which is provided with adhesive and/or the surface which is folded is disposed on at least one edge of the film or located there. This enables sterile closure of the film over an unsterile reusable apparatus in an easy manner without requiring a further closing means (e.g. rubber band or the like) and without any sticky interaction with the unsterile reusable apparatus. As a consequence of the closure, any contaminants can no longer pass from the unsterile reusable apparatus through the disposable apparatus, i.e. the unsterile reusable apparatus is closed in a seal relative to contaminants.

The film of the disposable apparatus can have at least one marking point which refers to an element of the unsterile reusable apparatus to be connected. With the help of a marking point, mounting of the disposable apparatus onto the unsterile reusable apparatus is simplified since checking the correct orientation of the adaptor on the disposable apparatus is superfluous.

The adaptor of the disposable device can be connected to the film via an adhesive connection and/or weld joint. It is important that the connection is in such a way that no contaminants (microorganisms and viruses) can pass the junction between the adaptor and the film.

The at least one anchoring point of the adaptor can protrude through the film from the upper side of the film to the underside of the film. In this embodiment, the film has an opening. It is hence crucial that, despite this opening, the junction between the film and the adaptor is sealed relative to contaminants. For example, this can be ensured by an adhesive connection and/or a weld joint.

In a preferred embodiment, the anchoring point comprises at least one electrical terminal which enables an electrical communication of the unsterile reusable apparatus with the sterile application device. The advantage hereby is that not only a mechanical communication between the reusable apparatus and the application device is possible (e.g. transfer of force from [a slide of] the reusable apparatus to [a syringe plunger of] the application device), but also that an electrical application device can be controlled directly (e.g. control of a cutting head of the application device via a rotary switch on the reusable apparatus).

The film can have a
a) length of 1 cm to 500 cm, preferably 10 cm to 200 cm, particularly preferably 20 cm to 100, in particular 40 cm to 80 cm; and/or
b) width of 1 cm to 500 cm, preferably 10 cm to 200 cm, particularly preferably 20 cm to 100, in particular 40 cm to 80 cm; and/or
c) thickness of 0.01 to 2 mm, preferably 0.02 to 1 mm, particularly preferably 0.05 to 0.8 mm, in particular 0.1 to 0.5 mm.

The advantage of a large length and width (i.e. a large surface extension) is that even a large unsterile reusable apparatus can be covered in a sterile manner. The advantageous of having a small thickness is that operating elements (e.g. buttons and/or controllers) of the reusable apparatus covered by the disposable apparatus can be seen and operated by the operator without difficulty. Moreover, small thicknesses provoke that the disposable apparatus can be produced with the least material expenditure and the volume and the weight of the disposable apparatus proves to be small.

The film can comprise polyurethane, polyacrylate, polyvinylchloride, polyethylene, polylactide, cellulose acetate and/or silicone or consist thereof. Polyethylene, polyvinylchloride and mixtures thereof are particularly preferred because they have self-adhesive properties, i.e. the film can be closed in a sealed manner relative to contaminants after covering the unsterile reusable apparatus, even without additional closing means.

Furthermore, a device is provided according to the invention for sterile application, comprising,
a) a disposable apparatus according to the invention;
b) a sterile application device;
c) an unsterile reusable apparatus comprising an electrical control unit for controlling the sterile application device;
or consisting thereof, characterised in that the upper side of the adaptor is connected to the sterile application device and the underside of the adaptor is connected to the unsterile reusable apparatus via the at least one anchoring point of the adaptor.

The sterile application device can comprise a liquid, preferably a liquid comprising cells, particularly preferably a liquid comprising skin cells, in particular a liquid comprising autologous skin cells.

The electrical control of the unsterile reusable apparatus is preferably suitable for controlling the application of liquid by the sterile application device. With such devices, it is possible to apply liquids and substances dissolved or suspended therein automatically, via an electrical control, on surfaces, such as e.g. human or animal skin.

The film can surround the space occupied by the unsterile reusable apparatus, at least partially, preferably completely. It is understood that a complete surrounding of the space occupied by the unsterile reusable apparatus means that the unsterile reusable apparatus is completely enclosed (encapsulated) by the film.

Preferably, the disposable apparatus of the device forms
a) via a self-adhesive surface of the film, via adhesive on a surface of the film and/or via a fold, preferably a fan-like fold; and/or
b) via a tensile band, preferably a rubber band, plastic band and/or adhesive band;
a closure sealed relative to the unsterile reusable apparatus for avoiding contamination of the sterile application device.

The sterile application device can comprise a spray head, a cutting head, a syringe and/or a spray solution container or consist thereof, preferably a spray head for surgical spray debridement equipment, a cutting head for surgical dermatomes, a disposable syringe and/or a spray solution container for surgical cell spraying equipment.

In a preferred embodiment, the unsterile reusable apparatus comprises at least one anchoring point for connecting to (and preferably fixing) the sterile application device, preferably a slide, the at least one anchoring point being suitable to transfer mechanical force to the sterile application device, preferably controlled by an electrical control unit.

In a further preferred embodiment, the unsterile reusable apparatus comprises at least one anchoring point suitable for establishing a locking connection to at least one anchoring point of the adaptor.

Also disclosed is a method for sterile covering of unsterile reusable apparatus is provided in addition, comprising the steps:
a) turning the film of the disposable apparatus according to the invention over an unsterile reusable apparatus so that the underside of the film is directed in the direction of the unsterile reusable apparatus and a space occupied by the unsterile reusable apparatus is surrounded completely by the film;
b) mechanical connection of the adaptor of the disposable apparatus to the unsterile reusable apparatus via the at least one anchoring point of the adaptor; and
c) air-tight closure of the film of the disposable apparatus.

In the case of the method according to the i disclosure, ;tep a) can be implemented before step b) or step b) before step a).

The subject according to the invention is intended to be explained in more detail with reference to the subsequent Figures and Examples, without wishing to restrict said subject to the specific embodiments represented here.

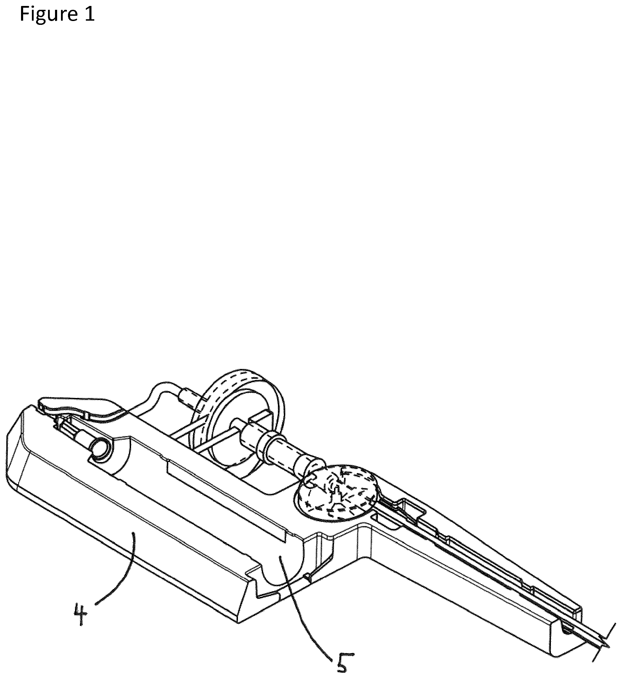

Figure 1 shows an illustration of a part of the inventive disposable apparatus, namely the adaptor 4 which is suitable for receiving a sterile application device. In this embodiment, the adaptor 4 is suitable for receiving the sterile application device because it comprises a mounting 5.

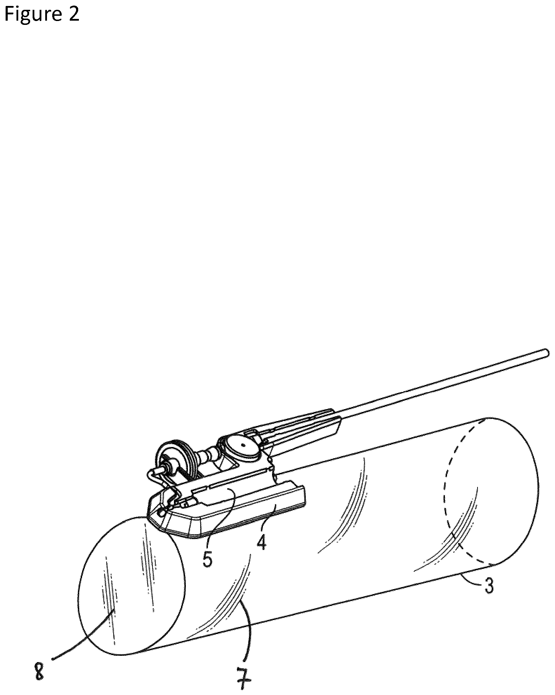

Figure 2 shows an illustration of an inventive disposable apparatus. The inventive disposable apparatus comprises a film 3 with a sterile upper side 7 and an underside 8. The adaptor 4 is suitable for receiving a sterile application device by connecting it to mounting 5.

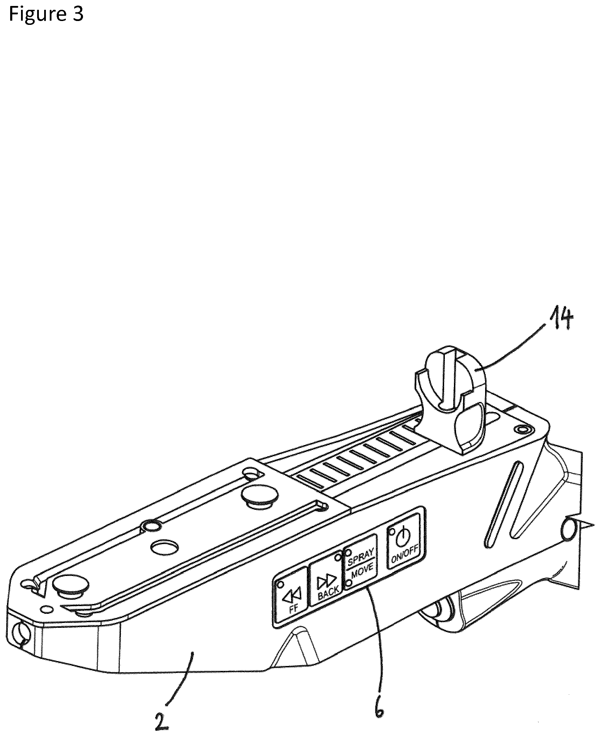

Figure 3 shows an illustration of an unsterile reusable apparatus 2 to which the inventive disposable apparatus may establish a reversible mechanical connection. In this embodiment, the unsterile reusable apparatus 2 is equipped with a slide 14 which is suitable to exert mechanical force on a sterile application device which is received by the inventive disposable apparatus. The unsterile reusable apparatus 2 comprises an electrical control unit 6 for controlling a sterile application device.

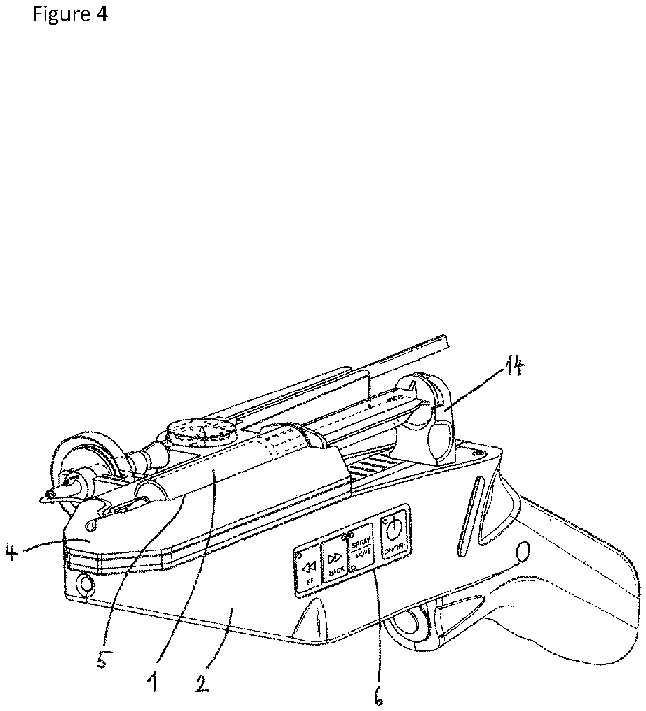

Figure 4 shows an illustration of an inventive device for sterile application. The inventive disposable apparatus forms a reversible mechanical connection to the unsterile reusable apparatus 2 via its adaptor 4 and the mounting 5 of the inventive disposable apparatus receives a sterile application device 1 (here: a syringe). The unsterile reusable apparatus 2 is equipped with a slide 14 which is suitable to exert mechanical force on the sterile application device 1 and comprises an electrical control unit 6 for controlling a sterile application device 1. The foil which is an essential part of the inventive disposable apparatus is omitted in this illustration for clarity reasons.

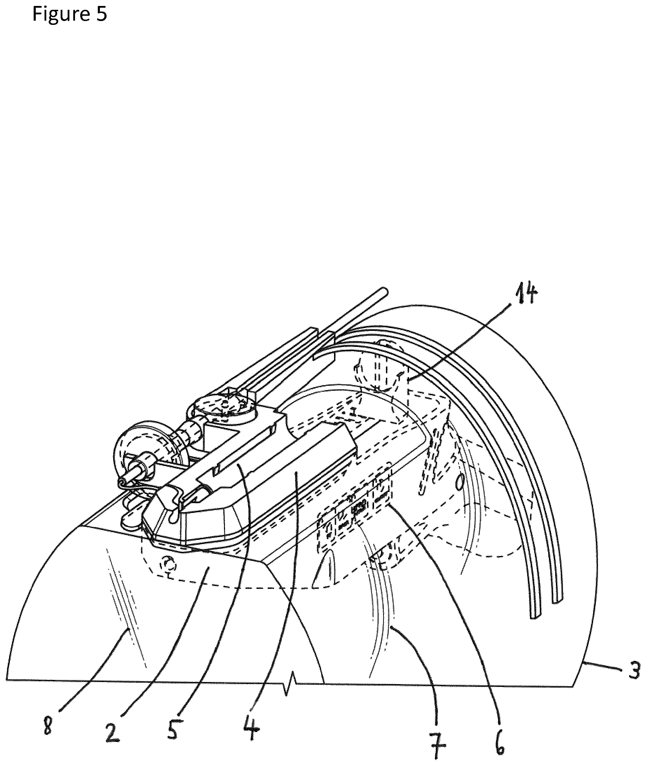

Figure 5 shows an illustration of a side view of an inventive device for sterile application. The inventive disposable apparatus is connected to an unsterile reusable apparatus 2 (unsterile reusable apparatus 2 is shown in dashed lines) and is in a configuration to receive a sterile application device 1. The unsterile reusable apparatus 2 is equipped with a slide 14 which is suitable to exert mechanical force on the sterile application device 1 cand omprises an electrical control unit 6 for controlling a sterile application device 1. The inventive disposable apparatus comprises a film 3 with a sterile upper side 7 and an underside 8. Furthermore, the disposable apparatus comprises at least one adaptor 4 with a sterile upper side and an underside, the sterile upper side of the adaptor 4 having a mounting 5 which is suitable for receiving a sterile application device 1. The underside of the adaptor 4 forms, with the upper side 7 of the film 3, an irreversible and air-tight connection for protection from contamination, i.e. the junction between the film 3 and the adaptor 4 represents a contamination barrier.

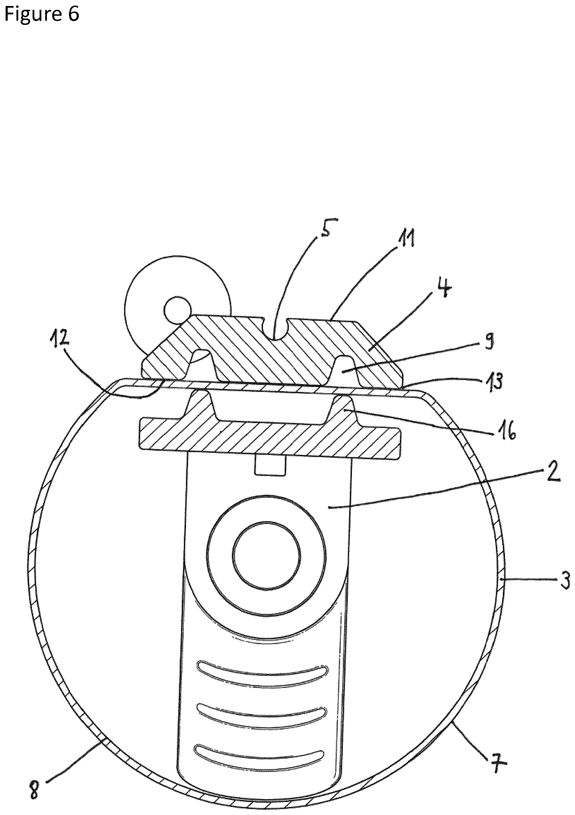

Figure 6 shows an illustration of a front view of an inventive device for sterile application. The inventive disposable apparatus is connected to an unsterile reusable apparatus 2 and is in a configuration to receive a sterile application device 1. The disposable apparatus comprises a film 3 with a sterile upper side 7 and an underside 8. Furthermore, the disposable apparatus comprises at least one adaptor 4 with a sterile upper side 11 and an underside 12, the sterile upper side 11 of the adaptor 4 having a mounting 5 which is suitable for receiving a sterile application device 1. The underside 12 of the adaptor 4 forms, with the upper side 7 of the film 3, an irreversible and air-tight connection 13 for protection from contamination, i.e. the junction between the film 3 and the adaptor 4 represents a contamination barrier. Furthermore, the disposable apparatus is characterised in that the underside 12 of the adaptor 4 has at least one anchoring point 9 for reversible mechanical connection to an anchoring point 16 of an unsterile reusable apparatus 2.

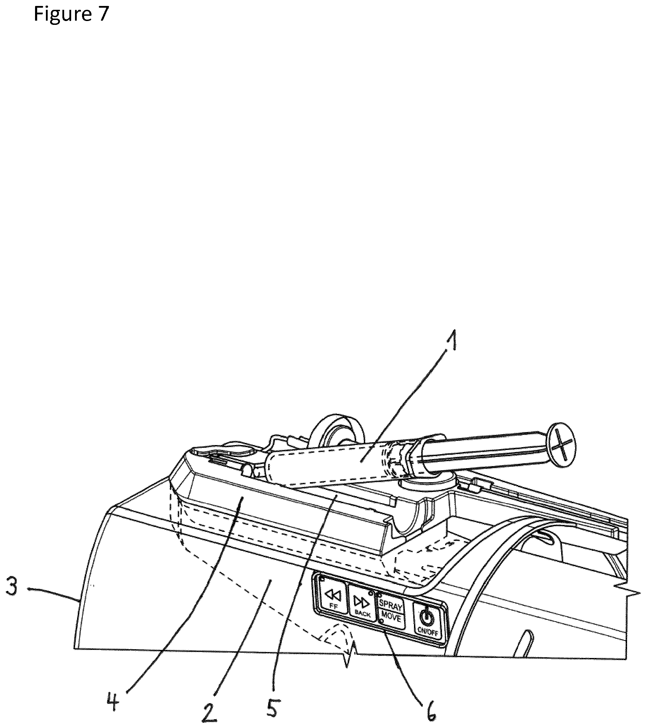

Figure 7 shows an illustration of an inventive device for sterile application, comprising a disposable apparatus according to the invention and an unsterile reusable apparatus 2. The unsterile reusable apparatus 2 comprises an electrical control unit 6 for controlling a sterile application device 1. The mounting 5 on the upper side of the adaptor 4 is configured to be connected to the sterile application device 1 (here: a syringe) and the underside of the adaptor 4 is connected via the at least one anchoring point of the adaptor 4 to the unsterile reusable apparatus 2.

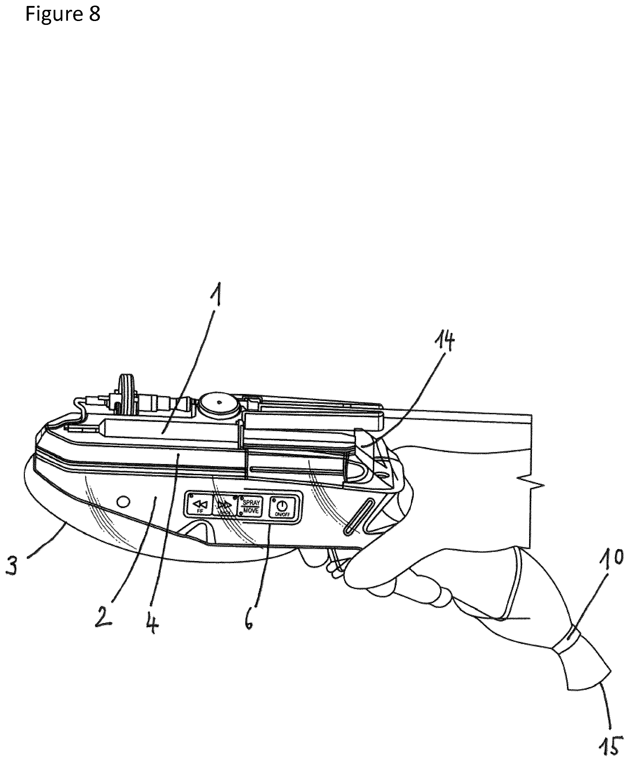

Figure 8 shows an illustration of an inventive device for sterile application, comprising an inventive disposable apparatus being connected to a sterile application device 1 (here: a syringe) and an unsterile reusable apparatus 2. The unsterile reusable apparatus 2 is equipped with a slide 14 which is suitable to exert mechanical force on the sterile application device 1, comprises an electrical control unit 6 for controlling a sterile application device 1 and is connected to the underside of the adaptor 4 via the at least one anchoring point of the adaptor 4. The film 3 of the disposable apparatus according to the invention surrounds the unsterile reusable apparatus 2 completely, the surrounded space being closed in an air-tight manner relative to the environment via a tensile band 10 (e.g. rubber band or adhesive band) acting in a constricting manner on the film 3. The air-tight closure via the tensile band 10 avoids contamination of the sterile application device 1 by the unsterile application device 2. In addition or alternative to the tensible band, the underside 8 of the film 3 may comprise a self-adhesive surface, an adhesive and/or a fan-fold at or next to at least one edge 15 of the film 3.

### Example - use of the disposable apparatus according to the invention

By way of example, a sterile-packaged disposable apparatus according to the invention is passed, in a surgical operating theatre, from "unsterile" assisting personnel to the "sterile" user (e.g. surgeon). The sterile packaging is then opened by the "sterile" user and the disposable apparatus is brought to the sterile operating table. Subsequently, the "unsterile" assisting personnel presents the unsterile reusable apparatus (e.g. an electrically operated irrigation sprayer) to the "sterile" user. The sterile user turns the disposable apparatus according to the invention, optionally already connected to a sterile application device via the adaptor, in such a way over the unsterile reusable apparatus that at least a portion, preferably every part, of the sterile application device is protected in a sterile manner by the film of the disposable apparatus. Subsequently, the user can operate the sterile application device of the invention and also operating elements on the reusable apparatus because of the small thickness of the film (e.g. actuation of the motor switch on the reusable apparatus through the film). In this way, the unsterile reusable apparatus (e.g. an irrigation sprayer) can be operated in a sterile manner by the user.

The invention is defined by the following claims.

## Claims

1. Disposable apparatus for sterile handling of an unsterile reusable apparatus (2), comprising
a film (3) with a sterile upper side (7) and an underside (8); and
at least one adaptor (4) with a sterile upper side (11) and an underside (12), wherein the sterile upper side (11) of the adaptor (4) is suitable for receiving a sterile application device (1) and wherein the underside (12) of the adaptor (4) has at least one anchoring point (9) for establishing a reversible mechanical connection to an unsterile reusable apparatus (2);
wherein the underside (12) of the adaptor (4) forms an irreversible and air-tight connection (13) with the upper side (7) of the film (3), **characterized in that** the adaptor (4) comprises a mounting (5) for a sterile application device (1) or consists thereof, wherein the mounting (5) is a syringe mounting and/or spray solution container mounting.

2. Disposable apparatus according to claim 1, **characterised in that** the mounting (5) is a syringe mounting for disposable syringes and/or a spray solution container mounting for surgical cell spraying equipment.

3. Disposable apparatus according to one of the preceding claims, **characterised in that** the film (3) has a flat configuration or tubular configuration, optionally has a tubular configuration and is closed on one side of the tube.

4. Disposable apparatus according to one of the preceding claims, **characterised in that** the film (3) has, at least in regions, on the upper side (7) and/or underside (8), a surface which
is self-adhesive; and/or
is provided with adhesive; and/or
is folded, preferably is folded like a fan;
wherein the self-adhesive surface, the surface which is provided with adhesive and/or the surface which is folded is/are preferably disposed at or next to at least one edge (15) of the film (3).

5. Disposable apparatus according to one of the preceding claims, **characterised in that** the film (3) has at least one marking point which refers to an element of the unsterile reusable apparatus (2) to be connected.

6. Disposable apparatus according to one of the preceding claims, **characterised in that** the adaptor (4) is connected to the film (3) via an adhesive connection (13) and/or weld joint (13).

7. Disposable apparatus according to one of the preceding claims, **characterised in that** the at least one anchoring point (9) of the adaptor (4) protrudes through the film (3) from the upper side (7) of the film (3) to the underside (8) of the film (3).

8. Disposable apparatus according to one of the preceding claims, **characterised in that** the anchoring point (9) comprises at least one electrical terminal which enables an electrical communication of the unsterile reusable apparatus (2) with the sterile application device (1).

9. Disposable apparatus according to one of the preceding claims, **characterised in that** the film (3) has a
length of 1 cm to 500 cm, preferably 10 cm to 200 cm, particularly preferably 20 cm to 100, in particular 40 cm to 80 cm;
width of 1 cm to 500 cm, preferably 10 cm to 200 cm, particularly preferably 20 cm to 100, in particular 40 cm to 80 cm; and/or thickness of 0.01 to 2 mm, preferably 0.02 to 1 mm, particularly preferably 0.05 to 0.8 mm, in particular 0.1 to 0.5 mm.

10. Disposable apparatus according to one of the preceding claims, **characterised in that** the film (3) comprises polyurethane, polyacrylate, polyvinylchloride, polyethylene, polylactide, cellulose acetate and/or silicone or consists thereof.

11. Device for sterile application, comprising
a) a disposable apparatus according to one of the preceding claims;
b) a sterile application device (1);
c) an unsterile reusable apparatus (2) comprising an electrical control unit (6) for controlling the sterile application device (1);
or consisting thereof, **characterised in that** the upper side (11) of the adaptor (4) is connected to the sterile application device (1) and the underside (12) of the adaptor (4) is connected to the unsterile reusable apparatus (2) via the at least one anchoring point (9) of the adaptor (4).

12. Device according to claim 11, **characterised in that** the sterile application device (1) comprises a liquid, preferably a liquid comprising cells, particularly preferably a liquid comprising skin cells, in particular a liquid comprising autologous skin cells.

13. Device according to one of the claims 11 or 12, **characterised in that** the electrical control unit (6) of the unsterile reusable apparatus (2) is suitable for controlling the application of liquid by the sterile application device (1).

14. Device according to one of the claims 11 to 13, **characterised in that** the film (3) surrounds the space occupied by the unsterile reusable apparatus (2), at least partially, preferably completely.

15. Device according to one of the claims 11 to 14, **characterised in that** the disposable apparatus forms
via a self-adhesive surface of the film (3), via adhesive on a surface of the film (3) and/or via a fold, preferably a fan-like fold; and/or
via a tensile band (10), preferably a rubber band, plastic band and/or adhesive band;
a closure sealed relative to the unsterile reusable apparatus (2) for avoiding contamination of the sterile application device (1).

16. Device according to one of the claims 11 to 15, **characterised in that** the sterile application device (1) comprises a spray head, a cutting head, a syringe and/or a spray solution container or consists thereof, preferably a spray head for surgical spray debridement equipment, a cutting head for surgical dermatomes, a disposable syringe and/or a spray solution container for surgical cell spraying equipment.

17. Device according to one of the claims 11 to 16, **characterised in that** the unsterile reusable apparatus (2) comprises at least one anchoring point (14) for the sterile application device (1), preferably a slide, the at least one anchoring point (14) being suitable to transfer mechanical force to the sterile application device (1), preferably controlled by an electrical control unit (6).

18. Device according to one of the claims 11 to 17, **characterised in that** the unsterile reusable apparatus (2) comprises at least one anchoring point (16) suitable for establishing a locking connection to at least one anchoring point (9) of the adaptor (4).

## Patentansprüche

1. Einweg-Vorrichtung zur sterilen Handhabung einer unsterilen Mehrweg-Vorrichtung (2), aufweisend
eine Folie (3) mit einer sterilen Oberseite (7) und einer Unterseite (8); und
zumindest einen Adapter (4) mit einer sterilen Oberseite (11) und einer Unterseite (12), wobei die sterile Oberseite (11) des Adapters (4) zur Aufnahme einer sterilen Applikationseinrichtung (1) geeignet ist und wobei die Unterseite (12) des Adapters (4) zumindest einen Verankerungspunkt (9) zur Herstellung einer reversiblen mechanischen Verbindung mit einer unsterilen Mehrweg-Vorrichtung (2) aufweist;
wobei die Unterseite (12) des Adapters (4) eine irreversible und luftdichte Verbindung (13) mit der Oberseite (7) des Films (3) bildet,
**dadurch gekennzeichnet, dass** der Adapter (4) eine Halterung (5) für eine sterile Applikationseinrichtung (1) aufweist oder daraus besteht, wobei die Halterung (5) eine Spritzenhalterung und/oder Sprühlösungsbehälterhalterung ist.

2. Einweg-Vorrichtung nach Anspruch 1, **dadurch gekennzeichnet, dass** die Halterung (5) eine Spritzenhalterung für Einweg-Spritzen und/oder eine Sprühlösungsbehälterhalterung für ein chirurgisches Zellsprühgerät ist.

3. Einweg-Vorrichtung nach einem der vorhergehenden Ansprüche,
**dadurch gekennzeichnet, dass** die Folie (3) eine flache Ausführung oder eine schlauchförmige Ausführung aufweist, optional eine schlauchförmige Ausführung aufweist und auf einer Seite des Schlauchs geschlossen ist.

4. Einweg-Vorrichtung nach einem der vorhergehenden Ansprüche,
**dadurch gekennzeichnet, dass** die Folie (3) zumindest bereichsweise auf der Oberseite (7) und/oder Unterseite (8) eine Oberfläche aufweist, die
selbstklebend ist; und/oder
mit Klebemittel versehen ist; und/oder
gefaltet ist, vorzugsweise wie ein Fächer gefaltet ist;
wobei die selbstklebende Oberfläche, die Oberfläche, die mit Klebemittel versehen ist, und/oder die Oberfläche, die gefaltet ist, vorzugsweise an oder nahe zumindest einer Kante (15) der Folie (3) angeordnet ist/sind.

5. Einweg-Vorrichtung nach einem der vorhergehenden Ansprüche, **dadurch gekennzeichnet, dass** die Folie (3) zumindest einen Markierungspunkt aufweist, der sich auf ein zu verbindendes Element der unsterilen Mehrweg-Vorrichtung (2) bezieht.

6. Einweg-Vorrichtung nach einem der vorhergehenden Ansprüche, **dadurch gekennzeichnet, dass** der Adapter (4) mit der Folie (3) über eine Klebeverbindung (13) und/oder Schweißnaht (13) verbunden ist.

7. Einweg-Vorrichtung nach einem der vorhergehenden Ansprüche, **dadurch gekennzeichnet, dass** der zumindest eine Verankerungspunkt (9) des Adapters (4) von der Oberseite (7) der Folie (3) zu der Unterseite (8) der Folie (3) durch die Folie (3) hindurch herausragt.

8. Einweg-Vorrichtung nach einem der vorhergehenden Ansprüche, **dadurch gekennzeichnet, dass** der Verankerungspunkt (9) zumindest einen elektrischen Anschluss umfasst, der eine elektrische Verbindung der unsterilen Mehrweg-Vorrichtung (2) mit der sterilen Applikationseinrichtung (1) ermöglicht.

9. Einweg-Vorrichtung nach einem der vorhergehenden Ansprüche, **dadurch gekennzeichnet, dass** die Folie (3) eine Länge von 1 cm bis 500 cm, vorzugsweise 10 cm bis 200 cm, besonders bevorzugt 20 cm bis 100, insbesondere 40 cm bis 80 cm;
eine Breite von 1 cm bis 500 cm, vorzugsweise 10 cm bis 200 cm, besonders bevorzugt 20 cm bis 100, insbesondere 40 cm bis 80 cm; und/oder
eine Dicke von 0,01 bis 2 mm, vorzugsweise 0,02 bis 1 mm, besonders bevorzugt 0,05 bis 0,8 mm, insbesondere 0,1 bis 0,5 mm; aufweist.

10. Einweg-Vorrichtung nach einem der vorhergehenden Ansprüche,
**dadurch gekennzeichnet, dass** die Folie (3) Polyurethan, Polyacrylat, Polyvinylchlorid, Polyethylen, Polylactid, Celluloseacetat und/oder Silikon enthält oder daraus besteht.

11. Einrichtung zur sterilen Applikation, aufweisend
a) eine Einweg-Vorrichtung gemäß einem der vorhergehenden Ansprüche;
b) eine sterile Applikationseinrichtung (1);
c) eine unsterile Mehrweg-Vorrichtung (2), die eine elektrische Steuerungseinheit (6) zum Steuern der sterilen Applikationseinrichtung (1) aufweist;
oder daraus bestehend, **dadurch gekennzeichnet, dass** die Oberseite (11) des Adapters (4) mit der sterilen Applikationseinrichtung (1) verbunden ist und die Unterseite (12) des Adapters (4) über den zumindest einen Verankerungspunkt (9) des Adapters (4) mit der unsterilen Mehrweg-Vorrichtung (2) verbunden ist.

12. Einrichtung nach Anspruch 11, **dadurch gekennzeichnet, dass** die sterile Applikationseinrichtung (1) eine Flüssigkeit enthält, vorzugsweise eine Flüssigkeit, die Zellen enthält, besonders bevorzugt eine Flüssigkeit, die Hautzellen enthält, insbesondere eine Flüssigkeit, die autologe Hautzellen enthält.

13. Einrichtung nach einem der Ansprüche 11 oder 12, **dadurch gekennzeichnet, dass** die elektrische Steuerungseinheit (6) der unsterilen Mehrweg-Vorrichtung (2) zum Steuern der Applikation von Flüssigkeit durch die sterile Applikationseinrichtung (1) geeignet ist.

14. Einrichtung nach einem der Ansprüche 11 bis 13, **dadurch gekennzeichnet, dass** die Folie (3) den durch die unsterile Mehrweg-Vorrichtung (2) belegten Raum zumindest teilweise, vorzugsweise komplett, umgibt.

15. Einrichtung nach einem der Ansprüche 11 bis 14, **dadurch gekennzeichnet, dass** die Einweg-Vorrichtung
über eine selbstklebende Oberfläche der Folie (3), über ein Klebemittel auf einer Oberfläche der Folie (3) und/oder über eine Falz, vorzugsweise eine fächerartige Falz; und/oder
über ein Zugband (10), vorzugsweise ein Gummiband, Plastikband oder Klebeband;
einen in Bezug auf die unsterile Mehrweg-Vorrichtung (2) abgedichteten Verschluss zur Vermeidung einer Kontamination der sterilen Applikationseinrichtung (1) bildet.

16. Einrichtung nach einem der Ansprüche 11 bis 15, **dadurch gekennzeichnet, dass** die sterile Applikationseinrichtung (1) einen Sprühkopf, einen Schneidkopf, eine Spritze und/oder einen Sprühlösungsbehälter, vorzugsweise einen Sprühkopf für ein chirurgisches Sprüh-Debridement-Gerät, einen Schneidkopf für chirurgische Dermatome, eine Einweg-Spritze und/oder einen Sprühlösungsbehälter für ein chirurgisches Zellsprühgerät, aufweist oder daraus besteht.

17. Einrichtung nach einem der Ansprüche 11 bis 16, **dadurch gekennzeichnet, dass** die unsterile Mehrweg-Vorrichtung (2) mindestens einen Verankerungspunkt (14) für die sterile Applikationseinrichtung (1), vorzugsweise einen Schlitten, aufweist, wobei der zumindest eine Verankerungspunkt (14) geeignet ist, mechanische Kraft auf die sterile Applikationseinrichtung (1) zu übertragen, vorzugsweise gesteuert durch eine elektrische Steuerungseinheit (6).

18. Einrichtung nach einem der Ansprüche 11 bis 17, **dadurch gekennzeichnet, dass** die unsterile Mehrweg-Vorrichtung (2) mindestens einen Verankerungspunkt (16) aufweist, der geeignet ist, eine Verriegelungsverbindung mit mindestens einem Verankerungspunkt (9) des Adapters (4) herzustellen.

## Revendications

1. Appareil jetable pour la manipulation stérile d'un appareil réutilisable non stérile (2), comprenant :
un film (3) avec un côté supérieur stérile (7) et une face inférieure (8) ; et
au moins un adaptateur (4) avec un côté supérieur stérile (11) et une face inférieure (12), dans lequel le côté supérieur stérile (11) de l'adaptateur (4) est approprié pour recevoir un dispositif d'application stérile (1) et dans lequel la face inférieure (12) de l'adaptateur (4) a au moins un point d'ancrage (9) pour établir un raccordement mécanique réversible sur un appareil réutilisable non stérile (2) ;
dans lequel la face inférieure (12) de l'adaptateur (4) forme un raccordement irréversible et étanche à l'air (13) avec le côté supérieur (7) du film (3),
**caractérisé en ce que** l'adaptateur (4) comprend un support (5) pour un dispositif d'application stérile (1) ou se compose de ce dernier, dans lequel le support (5) est un support de seringue et/ou un support de récipient de solution de pulvérisation.

2. Appareil jetable selon la revendication 1, **caractérisé en ce que** le support (5) est un support de seringue pour seringues jetables et/ou un support de récipient de solution de pulvérisation pour l'équipement de pulvérisation de cellule chirurgicale.

3. Appareil jetable selon l'une des revendications précédentes, **caractérisé en ce que** le film (3) a une configuration plate ou configuration tubulaire, facultativement a une configuration tubulaire et est fermé sur un côté du tube.

4. Appareil jetable selon l'une des revendications précédentes, **caractérisé en ce que** le film (3) a, au moins dans des régions, sur un côté supérieur (7) et/ou face inférieure (8), une surface qui :
est auto-adhésive ; et/ou
est pourvue d'un adhésif ; et/ou
est pliée, de préférence est pliée en accordéon ;
dans lequel la surface auto-adhésive, la surface qui est pourvue d'un adhésif et/ou la surface qui est pliée, est/sont de préférence disposée(s) au niveau de ou à proximité d'au moins un bord (15) du film (3).

5. Appareil jetable selon l'une des revendications précédentes, **caractérisé en ce que** le film (3) a au moins un point de marquage qui fait référence à un élément de l'appareil réutilisable non stérile (2) à raccorder.

6. Appareil jetable selon l'une des revendications précédentes, **caractérisé en ce que** l'adaptateur (4) est raccordé au film (3) via un raccordement adhésif (13) et/ou un joint de soudure (13).

7. Appareil jetable selon l'une des revendications précédentes, **caractérisé en ce que** le au moins un point d'ancrage (9) de l'adaptateur (4) fait saillie à travers le film (3) à partir du côté supérieur (7) du film (3) jusqu'à la face inférieure (8) du film (3).

8. Appareil jetable selon l'une des revendications précédentes, **caractérisé en ce que** le point d'ancrage (9) comprend au moins une borne électrique qui permet une communication électrique de l'appareil réutilisable non stérile (2) avec le dispositif d'application stérile (1).

9. Appareil jetable selon l'une des revendications précédentes, **caractérisé en ce que** le film (3) a :
une longueur de 1 cm à 500 cm, de préférence de 10 cm à 200 cm, en particulier de préférence de 20 cm à 100, en particulier de 40 cm à 80 cm ;
une largeur de 1 cm à 500 cm, de préférence de 10 cm à 200 cm, en particulier de préférence de 20 cm à 100, en particulier de 40 cm à 80 cm ; et/ou
une épaisseur de 0,01 à 2 mm, de préférence de 0,02 à 1 mm, en particulier de préférence de 0,05 à 0,8 mm, en particulier de 0,1 à 0,5 mm.

10. Appareil jetable selon l'une des revendications précédentes, **caractérisé en ce que** le film (3) comprend du polyuréthane, du polyacrylate, du polychlorure de vinyle, du polyéthylène, du polylactide, de l'acétate de cellulose et/ou de la silicone ou se compose de ces derniers.

11. Dispositif pour application stérile comprenant :
a) un appareil jetable selon l'une des revendications précédentes ;
b) un dispositif d'application stérile (1) ;
c) un appareil réutilisable non stérile (2) comprenant une unité de commande électrique (6) pour commander le dispositif d'application stérile (1) ;
ou se composant de ces derniers, **caractérisé en ce que** le côté supérieur (11) de l'adaptateur (4) est raccordé au dispositif d'application stérile (1) et la face inférieure (12) de l'adaptateur (4) est raccordée à l'appareil réutilisable non stérile (2) via le au moins un point d'ancrage (9) de l'adaptateur (4).

12. Dispositif selon la revendication 11, **caractérisé en ce que** le dispositif d'application stérile (1) comprend un liquide, de préférence un liquide comprenant des cellules, en particulier de préférence un liquide comprenant des cellules de peau, en particulier un liquide comprenant des cellules de peau autologues.

13. Dispositif selon l'une des revendications 11 ou 12, **caractérisé en ce que** l'unité de commande électrique (6) de l'appareil réutilisable non stérile (2) est apte à commander l'application de liquide par le dispositif d'application stérile (1).

14. Dispositif selon l'une des revendications 11 à 13, **caractérisé en ce que** le film (3) entoure l'espace occupé par l'appareil réutilisable non stérile (2), au moins partiellement, de préférence complètement.

15. Dispositif selon l'une des revendications 11 à 14, **caractérisé en ce que** l'appareil jetable forme :
via une surface autoadhésive du film (3), via l'adhésif sur une surface du film (3) et/ou via un pli, de préférence un pli en accordéon ; et/ou
via une bande extensible (10), de préférence une bande en caoutchouc, une bande en plastique et/ou une bande adhésive ;
une fermeture scellée par rapport à l'appareil réutilisable non stérile (2) pour éviter la contamination de l'appareil d'application stérile (1).

16. Dispositif selon l'une des revendications 11 à 15, **caractérisé en ce que** le dispositif d'application stérile (1) comprend une tête de pulvérisation, une tête de coupe, une seringue et/ou un récipient de solution de pulvérisation ou se compose de ces derniers, de préférence une tête de pulvérisation pour un équipement de débridement de pulvérisation chirurgical, une tête de coupe pour les dermatomes chirurgicaux, une seringue jetable et/ou un récipient de solution de pulvérisation pour l'équipement de pulvérisation de cellule chirurgicale.

17. Dispositif selon l'une des revendications 11 à 16, **caractérisé en ce que** l'appareil réutilisable non stérile (2) comprend au moins un point d'ancrage (14) pour le dispositif d'application stérile (1), de préférence une glissière, le au moins un point d'ancrage (14) étant apte à transférer la force mécanique au dispositif d'application stérile (1), de préférence commandé par une unité de commande électrique (6).

18. Dispositif selon l'une des revendications 11 à 17, **caractérisé en ce que** l'appareil réutilisable non stérile (2) comprend au moins un point d'ancrage (16) apte à établir un raccordement de verrouillage à au moins un point d'ancrage (9) de l'adaptateur (4).
